# EUROPEAN PATENT APPLICATION

(11) **EP 1 729 116 A1**
(43) Date of publication of application: **06.12.2006**
(21) Application number: 06113317.9
(22) Date of filing: 28.04.2006
(51) Int. Cl.: G01N 21/898, G01N 33/46, G01B 11/245, B27B 31/06, B07C 5/14

(54) **Process for photographically representing the outer appearance of a trunk and associating said photographic representation with the respective three-dimensional structure of the trunk and a device for implementing the procedure**

(30) Priority: 31.05.2005 IT BZ20050027
(71) Applicant: MICROTEC S.r.l., 39042 Bressanone (Bolzano) (IT)
(72) Inventor: Giudiceandrea, Federico, 39042, Bressanone (Bolzano) (IT)
(74) Representative: Ponchiroli, Simone

(57) **Abstract**

A process for photographically representing the outer appearance of a trunk (1) and associating said photographic representation with the respective three-dimensional structure of the trunk (1), comprises the following operating steps:
- light (3) is made to strike a section of trunk (1a), lighting up an approximately ring-shaped strip (4) of it consisting of its surface;
- two digital photographs of the strip (4) lit up are taken from two different viewpoints;
- the two photographs are photographically compared and composed;
- this digital photograph (1a) is associated point by point with the respective points of the three-dimensional structure of the section of trunk (1a).

## Description

The present invention relates to a process for photographically representing the outer appearance of a trunk 1 and associating said photographic representation with the respective three-dimensional structure of the trunk 1. The invention also relates to a device for implementing such a process.

Such a photographic representation is of interest in order to be able to assess the outer appearance of the tree trunk at a distance from it.

In turn, the outer appearance of a tree trunk is very closely linked to the quality of the timber which can be obtained from the trunk.

The quality of a trunk has always in the past and still is currently assessed visually, by careful inspection of the surface.

A plurality of parameters have to be considered when assessing the quality of a trunk. For example, the presence of fungi indicates rotten areas, whilst the presence of cracks, holes and the like may prevent the subsequent production of large pieces of timber.

If there are many knots or resin sacks, the quality of the wood is at least locally compromised and some types of machining may be prevented or made more difficult.

Even the shape of the trunk can provide information relating to the quality: straight trunks without bumps are preferred.

In the past this careful inspection was carried out by the buyer in the forest, immediately after the tree was cut down.

More recently, obviously this was not longer possible and the quality inspection on a trunk is carried out only by the seller, who guarantees the quality, in which case the buyer trusts this assessment and does not see the trunks he purchases, or only by the buyer, who inspects the trunks then reports to the seller, who is obliged to trust this assessment, or by both at the moment of purchase, although this involves the buyer going to the seller.

If an assessment proves false or incorrect, in addition to the evident economic damage, due to the fact that a buyer has paid more than the trunk is worth, there is further damage which is revealed only during machining and which consists in not being able to carry out the machining required because the wood does not meet the necessary quality requirements.

Since the assessment is visual, carried out by studying the surface of the trunk, the association of a trunk with an image of it is already known, in such a way that the quality assessment is performed by examining the photograph and not the surface of the trunk.

Obviously, the photograph must reliably represent the entire trunk, including both its length and circumference.

In an example of prior art relative to German patent DE 100 64 891 the association with a trunk of several electronic photographs representing its outer appearance is known, in such a way that on one hand said photographs may be sent by e-mail to a possible buyer or inserted in a catalogue on a CD-ROM, and on the other hand they may be kept in an electronic archive as documentation proving the quality attributed to the trunk or so that they can be retrieved from the archive at a later date if disputes arise.

To take the electronic photograph of the trunk, the known process involves photographing the trunk from above, along its longitudinal extension, lighting it up if necessary.

The corresponding device for implementing this process involves positioning a plurality of cameras above the trunk, evenly distributed along its longitudinal extension. Using these cameras, with the aid of any light sources needed to light up the trunk, a set of photographs is taken in series.

According to the known process, the photographs are taken simultaneously, probably with the trunk stationary. Each photograph relates to a section of trunk, in particular to the section of trunk underneath the respective camera of the device for implementing the process.

The document indicates that three cameras are sufficient to represent the entire length of a trunk, but obviously for particularly long trunks the fact that more cameras may be necessary cannot be ruled out. Moreover, the known process also involves photographing the two ends of the trunk, the front and the rear.

For this purpose the device for implementing the process comprises two additional cameras, for photographing at an angle the two ends of the trunk, where its cross-section is visible.

These two additional cameras are essential, since it is only through these that the inner part of the trunk can be represented, where the trunk has been cut and the growth rings are visible.

Therefore, to implement the known process at least five cameras are used, although the result achieved is only partial.

It should be considered that the known process allows only half of the trunk to be represented, since the cameras are positioned above the trunk. Only the upper part of the trunk is clearly represented, approximately one quarter of its entire curved extension, whilst as regards it sides the photograph is affected by the curvature of the trunk and its lower part is not photographed.

A knot or a rotten area located in the lower part of the trunk, the part resting on the conveyor device, is not photographed.

All of this is unsatisfactory.

To take detailed photographs of the entire trunk using said process, it would have to be photographed from four different viewpoints, arranged evenly all around the trunk, but that would mean using a large and unacceptable number of cameras, that is to say, fourteen, if a single camera were sufficient for each end, or even twenty, if four cameras also had to be located at each end of the trunk. If the trunk were very long, more cameras would be required on all four sides.

The purpose of the present invention is to develop an alternative process to the known one, which will make it possible to provide a reliable photographic representation of the outer appearance of the entire trunk.

This is achieved by a process with the characteristics indicated in the preamble to claim 1.

The present invention is based on the idea of photographically representing the trunk piece by piece, in particularly across its longitudinal direction and while it is fed along its longitudinal direction, then joining together the individual pieces and the relative photographs of them. All of this makes the process suitable for representing trunks of any length, without having to provide for local adaptation.

The characteristic claimed in claim 2 is a particularly reliable operating form of the process disclosed. The use of a plurality of light beams allows the strip lit up to be separated into individual portions, each of which is suitably lit up, so that by better lighting up the individual portion of surface its relative details can be clearly detected.

The characteristic claimed in claim 3 is an operating form of the process according to the invention which complements that claimed in claim 2. Separating the digital photography of the strip lit up into a set of digital photographs of the respective portions of surface lit up allows better overall photography of the strip lit up. It should also be noticed that the additional step of photographing each portion lit up twice, from opposite viewpoints, gives a further improvement of the photographic result which can be obtained, since the photograph resulting from the composition of these two photographs can contain the best of both.

The characteristic claimed in claim 4 allows an improved digital photograph of the two exposed cross-sections of the trunk to be obtained, that is to say those where the cut was made, since the additional lighting makes taking photographs easier and clearer.

Claims 6 and 5 relate to two alternative embodiments of the invention. The three-dimensional structure of the trunk to be covered with the appearance resulting from the digital photographs may also not be known beforehand, that is to say, the three-dimensional structure of the trunk to be subjected to the process disclosed may not be known. In such a case, in accordance with claim 5 during the operating step c) a comparison of the two digital photographs is also used to define the co-ordinates of each point of the section of trunk represented in the two digital photographs. Alternatively, according to claim 6 before the operating step a) the following preliminary operating step is implemented: the three-dimensional structure of the trunk is detected, so that the co-ordinates of each of its points are known.

As indicated, the invention also relates to a device for implementing the process disclosed, in particular a device which, using a suitable number of cameras, allows the entire trunk to be photographed.

This is achieved by a process with the characteristics indicated in the preamble to claim 7.

The invention is based on the idea that the trunk should be photographed across its longitudinal direction, photographing a plurality of sections of trunk while it is fed along its longitudinal direction. The trunk feed path is measured and this allows the different sections of trunk into which it is divided to be related to one another.

Claims 8 to 12 relate to embodiments of the device disclosed for implementing the operating forms of the process according to the invention claimed respectively in claims 2 to 6.

Further advantages and characteristics of the invention are more apparent in the description which follows of examples of operating forms of the process according to the invention and embodiments of the device disclosed, provided by way of example and without limiting the scope of the invention, with reference to the accompanying drawings, in which:
- Figure 1 is a schematic cross-section of the device according to the invention, shared by a first and second embodiment of the invention;
- Figure 2 is a schematic longitudinal section of a first embodiment of the device according to the present invention, at the moment when the trunk has just entered the lighting and photography zone;
- Figure 3 is a schematic longitudinal section of the device according to the present invention illustrated in Figure 1, at the moment when a generic section of the trunk is in the lighting and photography zone;
- Figure 4 is a schematic longitudinal section of the device according to the present invention illustrated in Figure 1, at the moment when the trunk is about to exit the lighting and photography zone;
- Figure 5 is a schematic longitudinal section of a second embodiment of the device according to the present invention, at the moment when a generic section of the trunk is in the lighting and photography zone.

As already indicated, the present invention relates to a process for photographically representing the outer appearance of a trunk 1 and associating said photographic representation with the respective three-dimensional structure of the trunk 1.

Such a process may be applied for example in assessing the quality of tree trunks to be sold to sawmills for producing timber.

In this sector, to keep things economical and simple to manage, it would be hoped that the certified quality of a trunk would also be verifiable afterwards or during machining of the trunk, when the structure of the trunk has already been altered.

The process disclosed allows all of this and the accompanying drawings show a device which allows implementation of this process.

The process relates to any trunk 1 which, being fed along its longitudinal direction, passes through a fixed lighting and photography zone 2 located across the direction of feed. As a consequence of this, during trunk 1 feed a section 1a, approximately cylindrical and always different of it, is located on each occasion in the lighting and photography zone 2.

The invention involves the following operating steps:
a) light 3, perpendicularly to the direction of feed, strikes the section of trunk 1a located on each occasion in the lighting and photography zone 2, lighting up the strip which is approximately ring-shaped 4 consisting of its surface;
b) two digital photographs of the strip 4 lit up are taken from two different viewpoints, located on the two opposite sides of the light 3 in the longitudinal direction;
c) the two digital photographs are compared and photographically composed to create a single photograph providing an unambiguous representation of the appearance of the section of trunk 1a;
d) this resulting digital photograph representing the appearance of the section of trunk 1a is associated point by point with the respective points of the three-dimensional structure of the section of trunk 1a, so as to cover the bare three-dimensional structure of the section of trunk 1a with the appearance resulting from this associated digital photograph, the joining of the three-dimensional structure and the associated appearance of all of these sections of trunk 1a constituting the overall three-dimensional structure and appearance of the trunk 1.

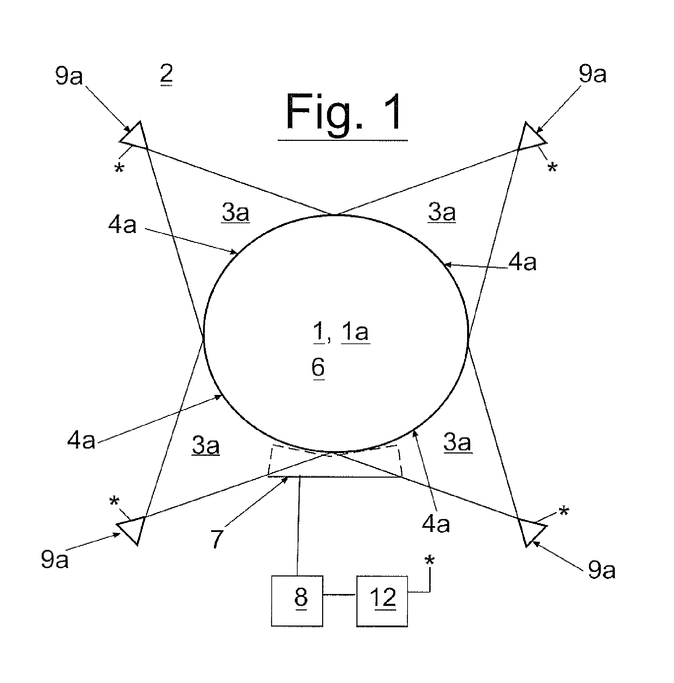

Appropriately, more in particular, during step a) of the process the light 3 consists of the set of a plurality of light beams 3a. Each light beam 3a lights up a portion 4a of the surface of the section of trunk 1a, therefore, the strip 4 lit up consists of the set of these portions 4a lit up. By way of example, in the accompanying drawings relative to the corresponding device for implementing the process, in particular Figure 1, there are four portions 4a lit up, on each occasion constituting the strip 4 lit up. This allows photography of the details of each portion 4a to be improved more than would occur if the entire strip 4 were to be photographed without separating it into portions.

To further improve the reliability of the photographs taken, during step b) of the process two digital photographs are taken of each portion 4a lit up. The optical axes according to which the digital photographs are taken are located on opposite sides of the light beam 3a. The two sets of digital photographs taken of the portions 4a lit up constitute the two digital photographs of the strip 4 lit up to be compared and composed. The digital photographs are easily managed using modern data transmission and printing systems.

Joining the three-dimensional structure and the associated appearance of all of these portions 4a constitutes the overall three-dimensional structure and appearance of the section of trunk 1a and subsequent joining of the three-dimensional structure and the associated appearance of all of these sections of trunk 1a constitutes the overall three-dimensional structure and appearance of the trunk 1.

The three-dimensional structure of the section of trunk 1a and the portion 4a may be defined, according to an operating form of the process, corresponding to a first embodiment of the corresponding device, in operating step c).

According to this, the comparison of the two digital photographs also allows the definition of the co-ordinates of each point of the section of trunk 1a represented in the two digital photographs. This is possible using the known triangulation technique.

Considering the two digital cameras as the vertices of a triangle whose other vertex is at the point photographed, it is possible to use trigonometry to find out all of the other measurements of this triangle, including the distance of the third vertex (the point photographed) from the base of the triangle, which is the important factor for identifying the co-ordinates of the point. The distance between the two digital cameras (base of the triangle) is known from the design and, because their value depends on the pixel which receives the light from the point photographed, the angles formed by the optical axes of the digital cameras with the base of the triangle can be identified. All of these assessments are made by the electronic processor 12.

Alternatively, the dimensional structure of the section of trunk 1a and the portion 4a may be defined, in accordance with another operating form of the process, corresponding to a second embodiment of the corresponding device, by including this preliminary step before operating step a) :
the three-dimensional structure of the trunk 1 is detected, so that the co-ordinates of all of its points are known. This is possible for example using a known scanner device 14, through which the trunk is passed.

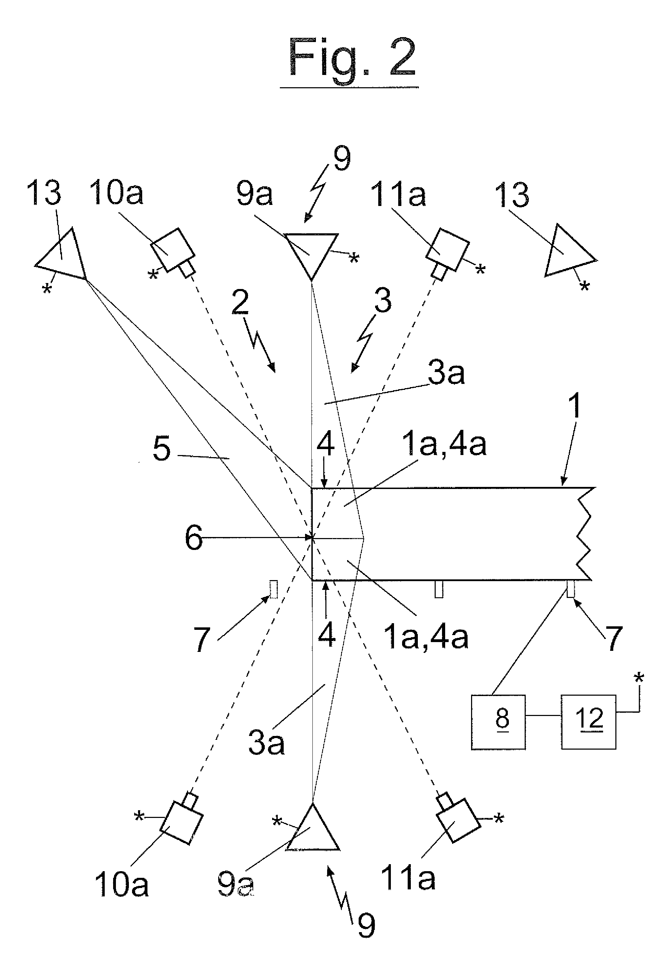

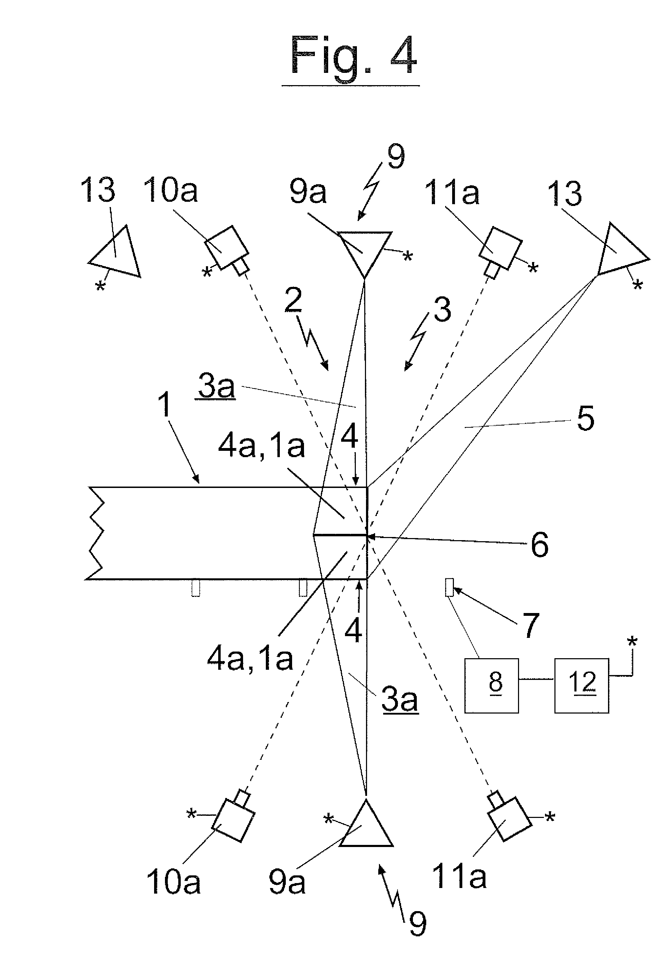

Finally, when the section of trunk 1a located in the lighting and photography zone 2 is the first or respectively the last of the set of sections of trunk 1a of which the trunk 1 consists, as illustrated in Figures 2 and respectively Figure 4, the light 3 and respectively the light beams 3a may not be enough to suitably light up the cross-section 6, both at the front and at the rear, made visible after the tree has been cut. To overcome this, the process disclosed requires that during operating step a) the front and rear of the section of trunk 1a are struck by additional light 5, so that the front or rear cross-section 6 is also well lit up.

As indicated, the invention also relates to a device for implementing the process.

Such a device comprises a measuring device 8, for measuring the trunk 1 feed path along the direction of feed. The trunk is fed forward by a conveyor medium 7 along its longitudinal direction.

There is a fixed lighting and photography zone 2. This is located across the direction of feed and, as the trunk 1 is fed forward, on each occasion there is always a different approximately cylindrical section 1a of the trunk in said zone.

The device disclosed also comprises a light source 9 and two digital photographic devices 10 and 11.

The light source 9, creates the light 3 which strikes, perpendicularly to the direction of feed, the section of trunk 1a on each occasion in the lighting and photography zone 2, lighting up an approximately ring-shaped strip 4 of it consisting of its surface. The two digital photographic devices 10, 11 are located in two different viewpoints, located on opposite sides of the light 3 in the longitudinal direction and each takes a digital photograph of the strip 4 lit up.

More specifically, appropriately, similarly to the corresponding operating form of the process disclosed, the light source 9 consists of the set of a plurality of light sources 9a. These are arranged evenly all around the measuring and photography zone 2, as illustrated in Figure 1 which by way of example shows four light sources 9a.

Each of these light sources 9a emits a light beam 3a which lights up an associated portion 4a of the surface of the section of trunk 1a. The set of these portions 4a lit up constitutes the strip 4 lit up.

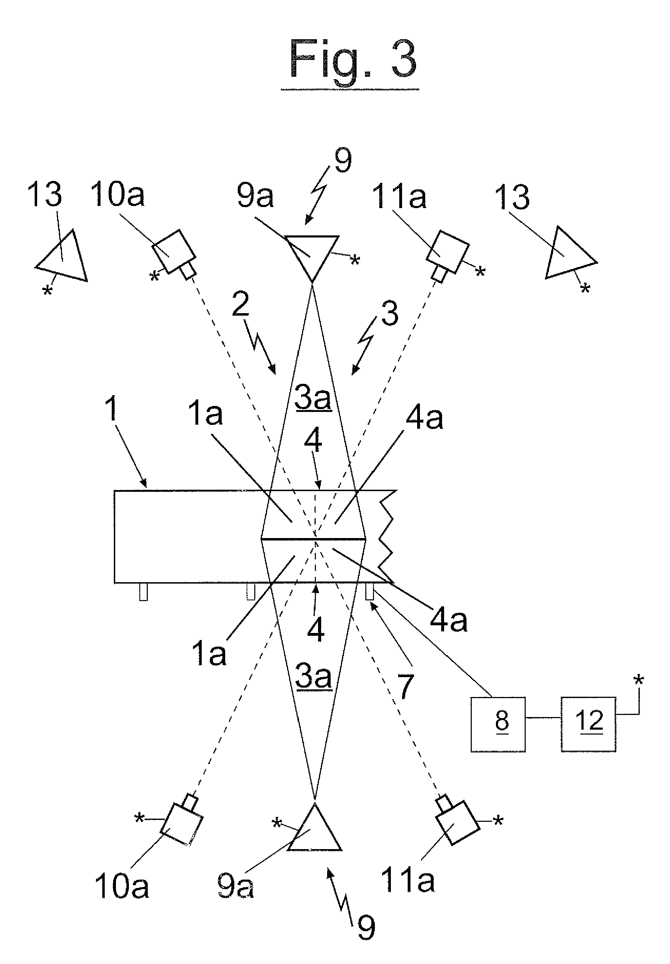

Moreover, similarly to the corresponding operating form of the process disclosed, the digital photographic devices 10, 11 consist respectively of a plurality of digital cameras 10a, 11a, in such a way that a pair of digital cameras 10a, 11a is associated with each portion 4a lit up. The optical axes of this pair of digital cameras 10a, 11a are located on opposite sides of the light beam 3a. In Figures 2 - 5 these axes are illustrated with a dashed line.

Each of the digital cameras 10a, 11a takes a digital photograph of the associated portion 4a lit up. The two sets of digital photographs taken of the portions 4a lit up constitute the two digital photographs of the strip 4 lit up to be compared and composed.

The device disclosed also comprises a processing unit 12, for comparing and photographically composing the two digital photographs taken by the digital photographic devices 10, 11, respectively by the digital cameras 10a, 11a and creating a resulting single digital photograph which unambiguously represents the section of trunk 1a.

All digital photograph manipulation takes place in this processing unit 12. In particular, this resulting digital photograph representing the appearance of the section of trunk 1a is associated point by point with the respective points of the three-dimensional structure of the section of trunk 1a, so as to cover the bare three-dimensional structure of the section of trunk 1a with the appearance resulting from this associated digital photograph.

Joining the three-dimensional structure and the associated appearance of all of these portions 4a constitutes the overall three-dimensional structure and appearance of the section of trunk 1a and subsequent joining of the three-dimensional structure and the associated appearance of all of these sections of trunk 1a constitutes the overall three-dimensional structure and appearance of the trunk 1.

According to a first embodiment of the device, relative to an operating form of the process, the electronic processor 12 also defines, in operating step c), the co-ordinates of each point of the section of trunk 1a represented in the two photographs. This is done by comparing the two digital photographs, as described with reference to the process.

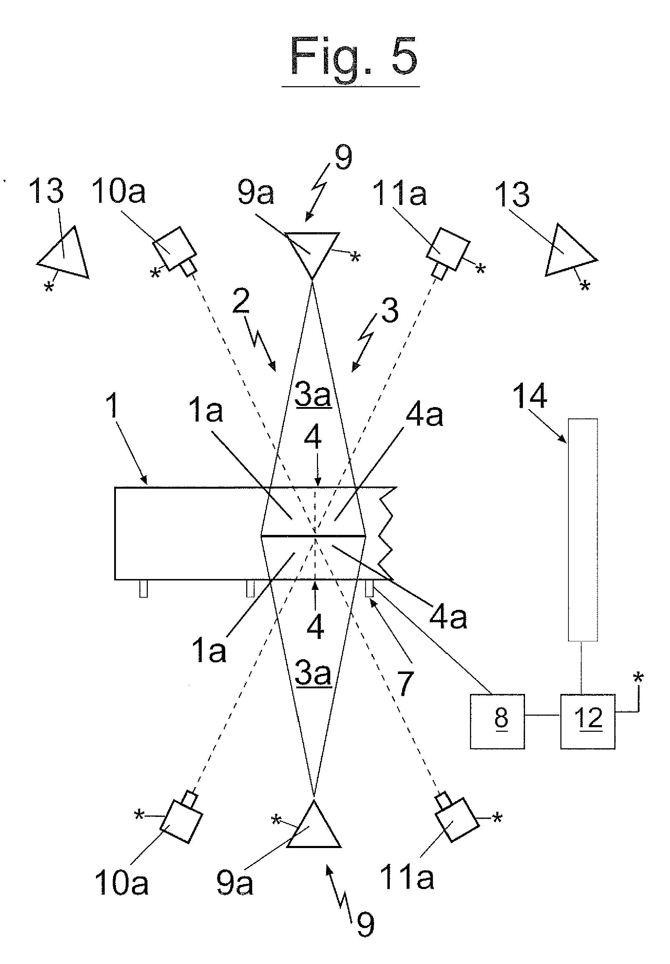

Alternatively, as illustrated in Figure 5, a second embodiment of the device also comprises a scanner device 14, for detecting the dimensional structure of the trunk 1 in such a way that the co-ordinates of each of its points are known. Such a scanner device 14 is known in various forms from the prior art.

When the section of trunk 1a located in the lighting and photography zone 2 is the first or respectively the last of the set of sections of trunk 1a of which the trunk 1 consists, as illustrated in Figures 2 and 4, according to the invention the device also comprises an additional light source 13, for creating additional light 5. In this way, light can be made to strike the front and rear of the section of trunk 1a, lighting up the front or respectively the rear cross-section 6 made visible after the tree has been cut, which otherwise might not be suitably lit up by the light 9 alone.

Known mathematical correlation techniques are used to manipulate the photographs in the process illustrated.

Each image in the photograph can be divided into a large number, which can be selected as required, of pixels, that is to say portions of image with uniform colour. For black and white images, each pixel is characterised by a more or less intense grey colour.

It should be noticed that the position of each pixel in the image is known and the intensity of the colour of the pixel can be associated with a number, which therefore mathematically represents the intensity.

Therefore, each pixel in a known position can have an associated number which depends on the intensity of its colour.

In turn, the image and therefore the photograph may be unambiguously characterised by the set of its pixels and precisely by the numbers associated with these pixels. Therefore, considering the numbers associated with the pixels in an image as co-ordinates in an n-dimensional space, in which n is equal to the number of pixels, in this n-dimensional space the image is unambiguously mathematically identifiable by this set of n-numbers.

In the same way parts of this image are unambiguously recognisable, for example when fully or partially overlapping images. Equally, said numerical comparison can be used to establish whether or not two images have parts common to both.

All of this is done using known analytical geometry calculation methods.

## Claims

1. A process for photographically representing the outer appearance of a trunk (1) and associating said photographic representation with the respective three-dimensional structure of the trunk (1), the trunk being fed along its longitudinal direction in such a way that it passes through a fixed lighting and photography zone (2) located across the direction of feed and during trunk (1) feed an approximately cylindrical and always different section (1a) of the trunk being located on each occasion in the lighting and photography zone (2), comprising the following operating steps:
a) light (3), perpendicularly to the direction of feed, strikes the section of trunk (1a) located on each occasion in the lighting and photography zone (2), lighting up the strip (4) which is approximately ring-shaped consisting of its surface;
b) two digital photographs of the strip (4) lit up are taken from two different viewpoints, located on the two opposite sides of the light (3) in the longitudinal direction;
c) the two digital photographs are compared and photographically composed to create a single photograph providing an unambiguous representation of the appearance of the section of trunk (1a);
d) this resulting digital photograph representing the appearance of the section of trunk (1a) is associated point by point with the respective points of the three-dimensional structure of the section of trunk (1a), so as to cover the bare structure of the section of trunk (1a) with the appearance resulting from this associated digital photograph, the joining of the structure and the associated appearance of all of these sections of trunk (1a) constituting the overall structure and appearance of the trunk (1).

2. The process according to claim 1, **characterised in that** in operating step a) the light (3) consists of the set of a plurality of light beams (3a), each light beam (3a) lighting up a portion (4a) of the surface of the section of trunk (1a) and the set of these portions (4a) lit up constituting the strip (4) lit up.

3. The process according to claim 2, **characterised in that** in operating step b) two digital photographs are taken of each portion (4a) lit up, the optical axes according to which the digital photographs are taken being located on opposite sides of the light beam (3a) and the two respective sets of digital photographs taken of the portions (4a) lit up constituting the two digital photographs of the strip (4) lit up to be compared and composed.

4. The process according to any of the foregoing claims from 1 to 3, **characterised in that** when the section of trunk (1a) located in the lighting and photography zone (2) is the first or respectively the last of the set of sections of trunk (1a) of which the trunk (1) consists, in operating step a) additional light (5) is made to strike the front and rear of the section of trunk (1a), lighting up the front and respectively the rear cross-section (6) made visible after the tree has been cut.

5. The process according to any of the foregoing claims from 1 to 4, **characterised in that** in operating step c) a comparison of the two digital photographs is also used to define the co-ordinates of each point of the section of trunk (1a) represented in the two digital photographs.

6. The process according to any of the foregoing claims from 1 to 4, **characterised in that** before operating step a) it comprises the preliminary operating step of:
detecting the three-dimensional structure of the trunk (1), so that the co-ordinates of each of its points are known.

7. A device for implementing a process for photographically representing the outer appearance of a trunk (1) and associating said photographic representation with the respective three-dimensional structure of the trunk (1), the trunk being fed by a conveyor medium (7) along its longitudinal direction in such a way that it passes through a fixed lighting and photography zone (2) located across the direction of feed and during trunk (1) feed an approximately cylindrical and always different section (1a) of the trunk being located on each occasion in the lighting and photography zone (2), comprising:
a measuring device (8), for measuring the trunk (1) feed path along the direction of feed;
a light source (9), for creating the light (3) which strikes, perpendicularly to the direction of feed, the section of trunk (1a) on each occasion in the lighting and photography zone (2), lighting up the approximately ring-shaped strip (4) of it consisting of its surface;
two digital photographic devices (10, 11), located in two different viewpoints, on opposite sides of the light (3) in the longitudinal direction, each taking a digital photograph of the strip (4) lit up;
a processing unit (12), for comparing and photographically composing the two digital photographs taken by the digital photographic devices (10, 11) to create a single digital photograph which unambiguously represents the appearance of the section of trunk (1a), where in this processing unit (12) this resulting digital photograph representing the appearance of the section of trunk (1a) is associated point by point with the respective points of the three-dimensional structure of the section of trunk (1a), so as to cover the bare structure of the section of trunk (1a) with the appearance resulting from this associated digital photograph, the joining of the structure and the associated appearance of all of these sections of trunk (1a) constituting the overall structure and appearance of the trunk (1).

8. The device according to claim 7, **characterised in that** the light source (9) consists of the set of a plurality of light sources (9a), each emitting a light beam (3a) which lights up a portion (4a) of the surface of the section of trunk (1a), the set of these portions (4a) lit up constituting the strip (4) lit up.

9. The device according to claim 8, **characterised in that** the digital photographic devices (10, 11) respectively consist of a plurality of digital cameras (10a, 11a), in such a way that a pair of digital cameras (10a, 11a) is associated with each portion (4a) lit up, the optical axes being located on opposite sides of the light beam (3a), each of the digital cameras (10a, 11a) taking a digital photograph of the associated portion (4a) lit up and the two respective sets of digital photographs taken of the portions (4a) lit up constituting the two digital photographs of the strip (4) lit up to be compared and composed.

10. The device according to any of the foregoing claims from 7 to 9, **characterised in that** it comprises an additional light source (13), for creating an additional light (5) with which, when the section of trunk (1a) located in the lighting and photography zone (2) is the first or respectively the last of the set of sections of trunk (1a) of which the trunk (1) consists, strikes the front and rear of the section of trunk (1a), lighting up the front or respectively the rear cross-section (6) made visible after the tree has been cut.

11. The device according to any of the foregoing claims from 7 to 10, **characterised in that** it also comprises a scanner (14), for detecting the three-dimensional structure of the trunk (1), in such a way that the co-ordinates of each of its points are known.

12. The device according to any of the foregoing claims from 7 to 10, **characterised in that** the processing unit (12) also uses a comparison of the two digital photographs to define the co-ordinates of each point of the section of trunk (1a) represented in the two digital photographs.
